# EUROPEAN PATENT APPLICATION

(11) **EP 4 793 369 A1**
(43) Date of publication of application: **19.08.2026**
(21) Application number: 24884879.8
(22) Date of filing: 31.10.2024
(51) Int. Cl.: C12Q 1/6886, C12N 15/113, A61P 35/00

(54) **USE OF GEMIN 4 GENE AND GEMIN 4 PROTEIN IN TUMOR**

(30) Priority: 31.10.2023 CN 202311429257
(71) Applicant: Nanjing Anji Biotechnology Co., Ltd., Nanjing, Jiangsu 210000 (CN)
(72) Inventor: XU, Hanmei, Nanjing, Jiangsu 210000 (CN)
(74) Representative: karo IP
(86) International application number: PCT/CN2024/128804
(87) International publication number: WO 2025/092879

(57) **Abstract**

This application discloses use of a *Gemin4* gene and a Gemin4 protein in a tumor, belongs to the technical field of biomedicine, and particularly relates to use of a *Gemin4* gene and a Gemin4 protein in the preparation of a reagent or medicament for detecting, preventing or treating a tumor, and use of a Gemin4 protein in the preparation of a reagent for detecting a tumor. The tumor includes one or more of human gliomas, liver cancer, colorectal cancer, esophageal cancer, kidney cancer, breast cancer, gastric cancer, head and neck cancer, bile duct cancer, and lung cancer.

## Description

### TECHNICAL FIELD

This application belongs to the technical field of biomedicine, and particularly relates to use of a *Gemin4* gene and a Gemin4 protein in tumors.

### BACKGROUND

Malignant tumors are the leading threat to human health. Unlimited growth, invasion and metastasis are hallmark features of malignancy, and are also the main causes of treatment failure and death. At present, the efficacy of medicaments is severely limited by the easy metastasis of tumors and the severe heterogeneity of tumor cells. Continuous exploration of the causes of tumors, the summary of tumor treatment and prevention plans, and the development of new and effective medicaments for treating tumors are crucial for clinical treatment of tumors.

Tumor markers are a group of substances that are produced by tumor cells *per se* or abnormally generated and/or increased due to the body's response to tumor cells in the occurrence and proliferation process of malignant tumors, and reflect the existence and/or growth of tumors. They include proteins, hormones, enzymes (isoenzymes), polyamines, and oncogene products, and the like, exist in the patient's blood, body fluids, cells or tissues, may be determined by biochemical methods, immunological methods, molecular biological methods, and the like, and have values in auxiliary diagnosis, differential diagnosis, therapeutic effect observation, recurrence monitoring and prognosis assessment of tumors. At present, markers available for precise tumor diagnosis are remain scarce. Identifying more effective tumor detection indicators and enhancing the auxiliary diagnosis value of the tumor markers are urgent problems to be solved.

Gemin4 (Gem Nuclear Organelle Associated Protein 4) is a key member of a GEMIN protein family and is used as an important component of a survival motor neuron (SMN) complex. It contributes to the integrity of a small ribonucleoprotein (snRNPs) structure by jointly binding the survival motor neuron protein (SMN) and GEMIN2, 3, 5, 6, 7, 8 in a complex to small nuclear RNA (snRNA), and the assembly of a spliceosome and the activation of downstream pre-mRNA is further promoted. Previous studies have shown that mutations in coding genes of SMN complex components are mainly closely associated with a motor neuron disease of spinal muscular atrophy (SMA). In addition, abnormal expression of Gemin5 and Gemin6 is associated with malignant phenotypes of various cancers. Recent studies have found that polymorphisms in the *GEMIN4* gene are associated with the development of colon cancer, bladder cancer, liver cancer, kidney cancer and ovarian cancer, but the expression level of the *GEMIN4* gene and the role of the expression of the Gemin4 protein in different tumors remain unclear.

### SUMMARY

### 1. Objects of the invention

The first objective of this application is to provide use of a *Gemin4* gene in the preparation of a tumor detection reagent or a medicament for preventing or treating tumors.

The second objective of this application is to provide use of a Gemin4 protein in preparation of a tumor detection reagent.

The third objective of this application is to provide use of a reagent for detecting the expression level of a *Gemin4* gene and a reagent for detecting the level of a Gemin4 protein in preparation of a tumor detection product.

The fourth objective of this application is to provide use of a reagent for knocking down or silencing the expression of a *Gemin4* gene in preparation of a medicament for preventing or treating tumors.

### 2. Technical solution

In order to achieve the above objectives of the invention, this application adopts the following technical solution:
This application provides use of a *Gemin4* gene in preparation of a tumor detection reagent or a medicament for preventing or treating tumors, and the nucleic acid sequence of the *Gemin4* gene is set forth in SEQ ID NO.1.

Further, the tumor includes one or more of human gliomas, liver cancer, colorectal cancer, esophageal cancer, kidney cancer, breast cancer, gastric cancer, head and neck cancer, bile duct cancer, and lung cancer.

Further, the *Gemin4* gene is highly expressed in human gliomas, liver cancer, or colorectal cancer.

Further, the *Gemin4* gene is underexpressed in human esophageal cancer, kidney cancer, breast cancer, gastric cancer, head and neck cancer, bile duct cancer, or lung cancer.

Further, the tumor detection reagent includes a specific primer pair with the nucleotide sequences set forth in SEQ ID NO.3 and SEQ ID NO.4.

Further, the above preventing or treating tumors is realized through knockdown or silencing of the expression of a *Gemin4* gene in tumor cells.

Further, the knockdown or silencing of the expression of the *Gemin4* gene is to design specific small interfering RNA designed according to the nucleotide sequence of the *Gemin4* gene, and transfect the specific small interfering RNA.

Further, the nucleotide sequence of the specific small interfering RNA is set forth in SEQ ID NO.5.

Further, the preventing or treating tumors includes inhibition of growth and/or metastasis of colorectal cancer, liver cancer and glioma cells.

Further, tumor cells of the tumor include one or more of the followings:
cancer cells of gliomas including: U87 and/or T98G;
cancer cells of colorectal cancer including: HT29; and
cancer cells of liver cancer including: HepG2.

This application further provides use of a Gemin4 protein in preparation of a tumor detection reagent. The amino acid sequence of the Gemin4 protein is set forth in SEQ ID NO.2.

Further, the tumor includes one or more of human head and neck cancer, lung cancer, gastric cancer, bile duct cancer, breast cancer, kidney cancer, esophageal cancer, colorectal cancer, liver cancer, and gliomas.

Further, the Gemin4 protein is highly expressed in human gliomas, liver cancer, or colorectal cancer.

Further, the Gemin4 protein is underexpressed in human esophageal cancer, kidney cancer, breast cancer, gastric cancer, head and neck cancer, bile duct cancer, or lung cancer.

This application further provides use of a reagent for detecting the expression level of a *Gemin4* gene in preparation of a tumor detection product.

Further, the tumor includes one or more of human gliomas, liver cancer, colorectal cancer, esophageal cancer, kidney cancer, breast cancer, gastric cancer, head and neck cancer, bile duct cancer, and lung cancer.

Further, the reagent for detecting the expression level of a *Gemin4* gene is a specific primer pair designed according to the *Gemin4* gene having the nucleotide sequence set forth in SEQ ID NO.1.

Further, the nucleotide sequences of the specific primer pair are set forth in SEQ ID NO.3 and SEQ ID NO.4.

Further, the *Gemin4* gene is highly expressed in human gliomas, liver cancer, or colorectal cancer.

Further, the *Gemin4* gene is underexpressed in human esophageal cancer, kidney cancer, breast cancer, gastric cancer, head and neck cancer, bile duct cancer, or lung cancer.

This application further provides use of a reagent for detecting the level of a Gemin4 protein in preparation of a tumor detection product.

Further, the tumor includes one or more of human gliomas, liver cancer, colorectal cancer, esophageal cancer, kidney cancer, breast cancer, gastric cancer, head and neck cancer, bile duct cancer, and lung cancer.

Further, the reagent for detecting the level of a Gemin4 protein includes a specific antibody of the Gemin4 protein, and means such as ELISA may be adopted for detection.

Further, the Gemin4 protein is highly expressed in human gliomas, liver cancer, or colorectal cancer.

Further, the Gemin4 protein is underexpressed in human esophageal cancer, kidney cancer, breast cancer, gastric cancer, head and neck cancer, bile duct cancer, or lung cancer.

This application further provides a tumor detection reagent kit, including the reagent for detecting the expression level of a *Gemin4* gene or the reagent for detecting the level of a Gemin4 protein.

Further, the tumor includes one or more of human gliomas, liver cancer, colorectal cancer, esophageal cancer, kidney cancer, breast cancer, gastric cancer, head and neck cancer, bile duct cancer, and lung cancer.

Further, the reagent for detecting the expression level of a *Gemin4* gene is a specific primer pair designed according to the *Gemin4* gene having the nucleotide sequence set forth in SEQ ID NO.1.

Further, the nucleotide sequences of the specific primer pair are set forth in SEQ ID NO.3 and SEQ ID NO.4.

Further, the *Gemin4* gene is highly expressed in human gliomas, liver cancer, or colorectal cancer.

Further, the *Gemin4* gene is underexpressed in human esophageal cancer, kidney cancer, breast cancer, gastric cancer, head and neck cancer, bile duct cancer, or lung cancer.

Further, the reagent for detecting the level of a Gemin4 protein includes a specific antibody of the Gemin4 protein, and means such as ELISA may be adopted for detection.

Further, the Gemin4 protein is highly expressed in human gliomas, liver cancer, or colorectal cancer.

Further, the Gemin4 protein is underexpressed in human esophageal cancer, kidney cancer, breast cancer, gastric cancer, head and neck cancer, bile duct cancer, or lung cancer.

This application further provides use of a reagent for knocking down or silencing the expression of a *Gemin4* gene in preparation of a medicament for preventing or treating tumors.

Further, the tumor includes one or more of gliomas, colorectal cancer, and liver cancer.

Further, the above preventing or treating tumors is realized through knockdown or silencing of the expression of a *Gemin4* gene in tumor cells.

Further, the knockdown or silencing of the expression of the *Gemin4* gene is to design specific small interfering RNA designed according to the nucleotide sequence of the *Gemin4* gene.

Further, the reagent for knocking down or silencing the expression of the *Gemin4* gene includes specific siRNA for the *Gemin4* gene.

Further, the reagent for knocking down or silencing the expression of the *Gemin4* gene includes siRNA with the nucleotide sequence set forth in SEQ ID NO.5.

Further, the preventing or treating tumors includes inhibition of growth and/or metastasis of tumor cells.

Further, tumor cells of the tumor include one or more of the followings:
cancer cells of gliomas including: U87 and/or T98G;
cancer cells of colorectal cancer including: HT29; and
cancer cells of liver cancer including: HepG2.

This application further provides a pharmaceutical composition for preventing or treating a tumor. The pharmaceutical composition at least includes a reagent for knocking down or silencing the expression of a *Gemin4* gene and a pharmaceutically acceptable carrier.

Further, the tumor includes one or more of gliomas, colorectal cancer, and liver cancer.

Further, the reagent for knocking down or silencing the expression of the *Gemin4* gene includes specific siRNA for the *Gemin4* gene.

Further, the reagent for knocking down or silencing the expression of the *Gemin4* gene includes siRNA with the nucleotide sequence set forth in SEQ ID NO.5.

Further, the preventing or treating tumors includes inhibition of growth and/or metastasis of tumor cells.

Further, tumor cells of the tumor include one or more of the followings:
cancer cells of gliomas including: U87 and/or T98G;
cancer cells of colorectal cancer including: HT29; and
cancer cells of liver cancer including: HepG2.

### 3. Beneficial effects

Compared with the prior art, this application has the following beneficial effects:
(1) In this application, the expression levels of a *Gemin4* gene in tumor cells and normal cells are detected by a quantitative real time PCR method. It has been found that the expression level of the *Gemin4* gene may be significantly decreased in human esophageal cancer cells TE13 and Eca109, kidney cancer cells A498, breast cancer cells MDA-MB-231, gastric cancer cells MGC803, head and neck cancer cells CAL27, bile duct cancer cells RBE and HuCC-T1, and lung cancer cells A549, and the expression level may be significantly increased in human glioma cells T98G and U87, liver cancer cells HepG2, and colorectal cancer cells HT29. The above findings indicate that the Gemin4 gene may be used as a potential tumor marker for auxiliary diagnosis of early-stage tumors.
(2) In this application, based on the verification of the expression level of a *Gemin4* gene in the tumor cells by quantitative real time PCR, immunoblotting detection is performed by using specific antibodies against the Gemin4 protein, the results are found to be consistent with those of qPCR, the expression level of the Gemin4 protein may be significantly decreased in human esophageal cancer cells TE13 and Eca109, kidney cancer cells A498, breast cancer cells MDA-MB-231, gastric cancer cells MGC803, head and neck cancer cells CAL27, bile duct cancer cells RBE and HuCC-T1, and lung cancer cells A549, and the expression level may be significantly increased in human glioma cells T98G and U87, liver cancer cells HepG2, and colorectal cancer cells HT29. The above findings indicate that the Gemin4 protein may be used as a potential tumor marker for auxiliary diagnosis of early-stage tumors.
(3) In this application, specific siRNAs targeting the nucleotide sequence encoding the Gemin4 protein are designed for the human glioma cells T98G and U87, liver cancer cells HepG2, and colorectal cancer cells HT29 in which the expression level of the *Gemin4* gene is significantly increased., Transfection of these cancer cells significantly inhibits the growth and/or metastasis of glioma, liver cancer and colorectal cancer cells. This indicates that the targeted inhibition (knockdown or silencing) of the *Gemin4* gene can be used for treating gliomas, liver cancer and colorectal cancer.

### BRIEF DESCRIPTION OF THE DRAWINGS

FIG. 1 shows significantly downregulated expression of a *Gemin4* gene in different tumor cells, as detected by qPCR.
FIG. 2 shows significantly upregulated expression of a *Gemin4* gene in different tumor cells, as detected by qPCR.
FIG. 3 shows significantly downregulated expression of a Gemin4 protein in different tumor cells, as detected by Western blot.
FIG. 4 shows significantly upregulated expression of a Gemin4 protein in different tumor cells, as detected by Western blot.
FIG. 5 is RNA and protein level expression of a *Gemin4* gene after si-Gemin4 transfection.
FIG. 6 shows an inhibitory effect of *Gemin4* gene knockdown on tumor cell proliferation.
FIG. 7 shows an inhibitory effect of *Gemin4* gene knockdown on tumor cell migration.

### DETAILED DESCRIPTION

This application will be further described hereafter in conjunction with specific examples.

It should be noted that terms such as "up", "down", "left", "right", and "middle" cited in this specification are only used for the sake of clarity in description and are not intended to limit the scope of implementation. Any changes or adjustments in their relative relationships, without substantial alterations to the technical content, shall also be regarded as within the scope of implementation of this application.

Unless otherwise defined, all technical and scientific terms used in this application have the same meanings as those usually understood by those skilled in the art to which this application belongs. The terms "and/or" used in this application include any and all combinations of one or more related listed items.

Where no specific conditions are specified in the examples, the conventional conditions or the conditions recommended by the manufacturer shall be followed. The reagents or instruments used without a manufacturer indicated are all conventional products that are commercially available.

As used in this application, the term "about" is used to provide flexibility and imprecision associated with a given term, measure, or value. Those skilled in the art may easily determine the degree of flexibility of specific variables.

As used in this application, the term "at least one of..." is intended to be synonymous with "one or more of ...". For example, "at least one of A, B and C" explicitly includes only A, only B, only C and their respective combinations.

The concentration, quantity and other numerical data may be presented in a range format in this application. It should be understood that such a range format is only used for convenience and conciseness, and should be flexibly interpreted as not only including the values explicitly described as the limit of the range, but also all individual values or subranges covered within the range, as if each value and subrange are explicitly described. For example, a numerical range of about 1 to about 4.5 should be interpreted as not only including the explicitly stated limit values of 1 to about 4.5, but also including individual numbers (such as 2, 3, and 4) and subranges (such as 1 to 3, and 2 to 4). The same principle is applicable to ranges that describe only one value. For example, "less than about 4.5" should be interpreted as including all the aforementioned values and ranges. Furthermore, this interpretation should be applicable regardless of the described scope or the breadth of the features.

### Example 1

In this example, the expression levels of a *Gemin4* gene in tumor cells and normal cells were detected by quantitative real time PCR .

The tumor cells included:
human microglial cells HMC3, and human glioma cells T98G and U87;
human liver cells L-02, and liver cancer cells HepG2;
colorectal cancer cells HT29;
human normal esophageal epithelial cells HEEC, and esophageal cancer cells TE13 and Eca109;
human kidney epithelial cells HEK293T, and kidney cancer cells A498:
   human normal mammary epithelial cells MCF10A, and breast cancer cells MDA-MB-231;
   gastric cancer cells MGC803;
   head and neck cancer cells CAL27;
   bile duct cancer cells RBE and HuCCT1; and
   lung cancer cells A549.

A detection method included:

### (1) Total RNA extraction in tumor cells and normal epithelial cells

The above 18 kinds of cells were respectively cultured at 37°C with 5% CO₂ in an incubator. The cells were trypsinized when cultured to reach a density of 90%, and were collected. The cells were resuspended by a culture solution, and were counted under a microscope. The concentration of the cells was adjusted to 5×10⁵ cells/mL, and then, a cell suspension after concentration adjustment was seeded into a 6-well plate at a dose of 2 mL per well, and was continuously cultured for 24 h in the 37°C 5% CO₂ incubator.

The total RNA of the cells was respectively extracted according to TRIzol instructions of Tiangen Biochemical Technology (Beijing) Co., LTD. Then, the purity and concentration of the extracted RNA were quantified by using a NanoDrop ND-1000 nucleic acid quantifier. Agarose quality inspection was performed to ensure the integrity of the extracted RNA.

### (2) Synthesis of first-strand cDNA through RNA reverse transcription

The extracted total RNA was subjected to reverse transcription by using an ABM reagent kit PrimeScript TM RT reagent Kit with gDNA Eraser (Perfect Real Time) to synthesize cDNA. This reagent kit included gDNA Eraser DNase, and mixed genomic DNA may be effectively removed.

### (3) Real-time quantitative PCR

A specific primer was designed according to the nucleotide sequences of the *Gemin4* gene and the *GAPDH* gene. An ABM reagent kit SYBR^{®} Premix Ex Taq^{™} II (TliRNaseH Plus) was used for qPCR reaction, the sequences of a forward primer and a reverse primer of the *Gemin4* were respectively set forth in SEQ ID NO.3 and SEQ ID NO.4, and the sequences of a forward primer and a reverse primer of the *GAPDH* were respectively set forth in SEQ ID NO.6 and SEQ ID NO.7. A reaction system was as shown in Table 1:

**Table 1 PCR Reaction System**

| Reagent | Dose (µL) |
|---|---|
| SYBR Premix Ex Taq II(TliRNaseH Plus)(2×) | 12.5 |
| PCR Forward Primer (10 µM) | 1 |
| PCR Reverse Primer (10 µM) | 1 |
| DNA template (<100 ng) | 2 |
| Sterile water | 8.5 |
| Total | 25 |

After uniformly mixed, the above ingredients were subjected to real-time quantitative PCR: 95°C 30 s pre-denaturation, 40 cycles; and 95°C 5 s, and 60°C 30 s according to the following procedures.

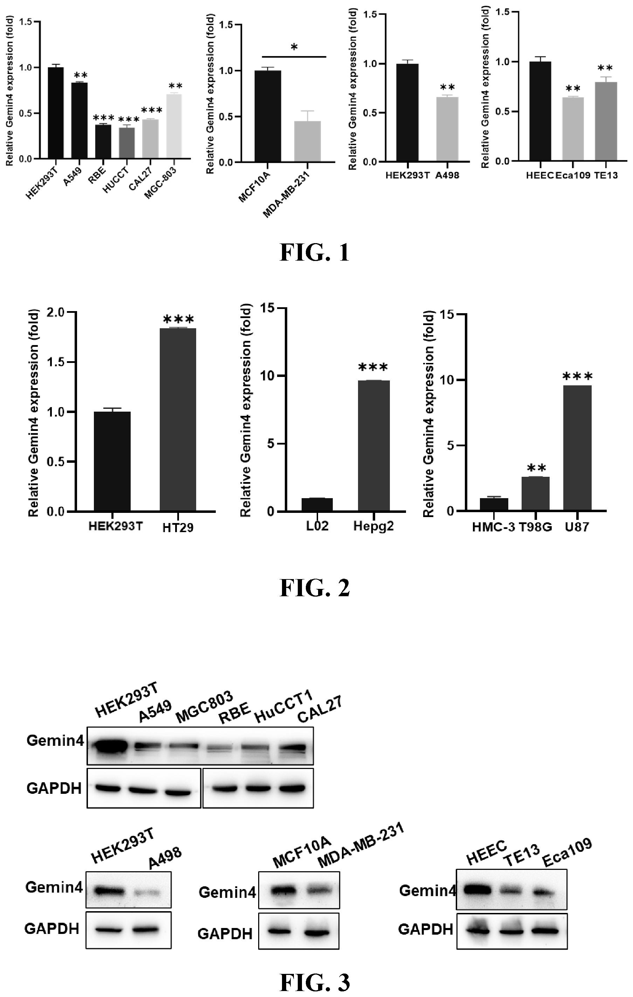

The specificity of the reaction was determined based on a melting curve, and the expression level of the *Gemin4* gene was calculated by Formula 2^{-ΔΔCt}. The results are as shown in FIG. 1 and FIG. 2. FIG. 1 shows that in comparison to that in human normal cells, the expression levels of the *Gemin4* gene in human esophageal cancer cells TE13 and Eca109, kidney cancer cells A498, breast cancer cells MDA-MB-231, gastric cancer cells MGC803, head and neck cancer cells CAL27, bile duct cancer cells RBE and HuCC-T1, and lung cancer cells A549 are significantly decreased, and FIG. 2 shows that in comparison to that in human normal cells, the expression level of the *Gemin4* gene is significantly increased in human glioma cells T98G and U87, liver cancer cells HepG2, and colorectal cancer cells HT29.

### Example 2

In this example, the expression levels of the Gemin4 protein in tumor cells and normal cells were detected by a Western Blot method.

The tumor cells included:
human microglial cells HMC3, and human glioma cells T98G and U87;
human liver cells L-02, and liver cancer cells HepG2;
colorectal cancer cells HT29;
human normal esophageal epithelial cells HEEC, and esophageal cancer cells TE13 and Eca109;
human kidney epithelial cells HEK293T, and kidney cancer cells A498:
   human normal mammary epithelial cells MCF10A, and breast cancer cells MDA-MB-231;
   gastric cancer cells MGC803;
   head and neck cancer cells CAL27;
   bile duct cancer cells RBE and HuCCT1; and
   lung cancer cells A549.

A detection method included:
protein extraction in tumor cells and normal epithelial cells:
The above 18 kinds of cells were respectively cultured at 37°C with 5% CO₂ in an incubator. The cells were trypsinized when cultured to reach a density of 90%, and were collected. The cells were resuspended by a culture solution, and were counted under a microscope. The concentration of the cells was adjusted to 5×10⁵ cells/mL, and then, a cell suspension after concentration adjustment was seeded into a 6-well plate at a dose of 2 mL per well, and was continuously cultured for 24 h at 37°C with 5% CO₂ incubator.

After centrifugation, a supernatant was discarded. Rinsing was performed twice with PBS, and a supernatant was discarded. A RIPA lysate was added to perform lysing on ice for 20 min. A supernatant was collected after centrifugation at 12000 g for 10 min. A 1×SDS sample loading buffer was added. After uniform mixing through by pipetting, denaturation was performed by boiling for 5 min. A total protein was separated by 10% SDS-PAGE gel, and was then transferred to a PVDF membrane. The membrane was blocked with 5% BSA at room temperature for 2 h, was incubated overnight with a GAPDH antibody and a Gemin4 antibody at 4°C, and washed three times with TBST. The membranes were incubated with a second antibody at room temperature for 1 h, and washed three times with TBST. Protein bands were visualized using an ECL chemiluminescent substrate and detected with a Tanon imaging system.

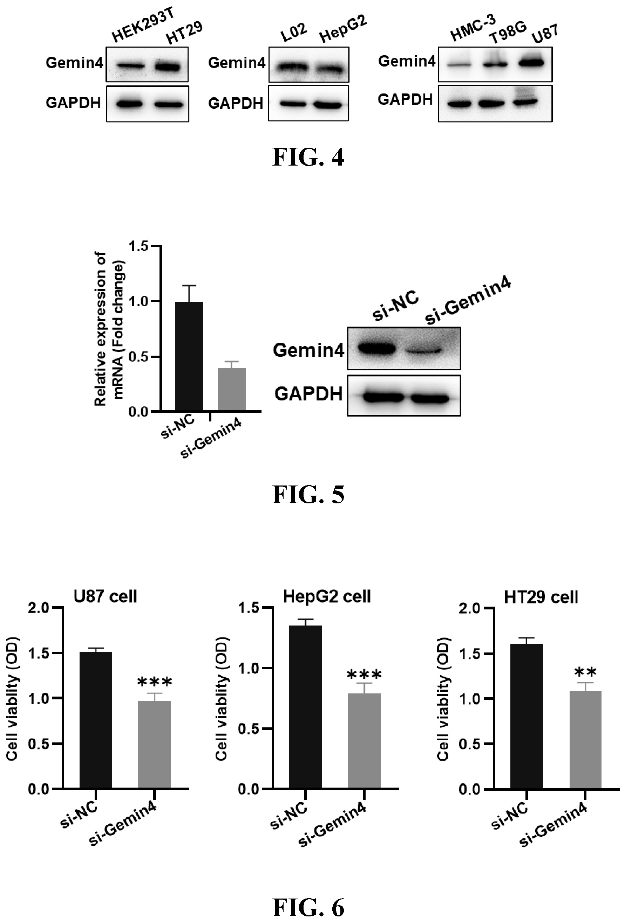

The results are as shown in FIG. 3 and FIG. 4. FIG. 3 shows that in comparison to that in human normal cells, the expression levels of the Gemin4 protein in human esophageal cancer cells TE13 and Eca109, kidney cancer cells A498, breast cancer cells MDA-MB-231, gastric cancer cells MGC803, head and neck cancer cells CAL27, bile duct cancer cells RBE and HuCC-T1, and lung cancer cells A549 are significantly decreased, and as shown in FIG. 4, FIG. 4 shows that in comparison to that in human normal cells, the expression level of the Gemin4 protein is significantly increased in human glioma cells T98G and U87, liver cancer cells HepG2, and colorectal cancer cells HT29. The above results are consistent with the qPCR results in Example 1, and it indicates that Gemin4 can be used as a potential marker for tumor detection.

### Example 3

This example provides the influence of silencing the expression of the *Gemin4* gene on the cell proliferation ability of tumor cells.

Specific siRNA was designed for the nucleotide sequence of the *Gemin4* gene, with the specific sequence set forth in SEQ ID NO.5 (GCUCCUGUGUGAGAUUGUATT). Glioma cells U87, colorectal cancer cells HT29, and liver cancer cells HepG2 were respectively cultured at 37°C with 5% CO₂ in an incubator. The cells were digested 24 h in advance, was laid in a 12-well plate at a density of 8×10⁴ cells /mL, and cell transfection was performed when the cell confluence reached 60%. In a 1.5 mL sterile EP tube, 50 µL of a DMEM medium was added, followed by 8 µL of a transfection reagent. Uniform mixing was performed by pipetting, followed by still standing at room temperature for 5 min. In another two EP tubes, 50 µL of a DMEM medium was added, followed by 6 µL of siRNA (si-Gemin4 and si-NC, the nucleotide sequence of the si-NC was set forth in SEQ ID NO.8: ACGUGACACGUUCGGAGAATT). Uniform mixing was performed by pipetting, followed by still standing for 5 min. A transfection reagent mixture obtained in the first step was dropwise added to the second EP tube. Uniform mixing was performed by pipetting, and incubation was performed at room temperature for 20 min. During the incubation, the original culture medium in the 6-well plate was aspirated and replaced with a fresh complete culture medium. After the incubation was completed, the transfection mixture was dropwise added into the 6-well plate, uniform mixing was performed through shaking, the mixture was put into an incubator for culture, the medium was changed after 6 h, and digestion and functional assays were carried out after 48 h.

The two groups of transfected cells were cultured at 37°C with 5% CO₂ in an incubator. The cells were then trypsinized when cultured to reach a density of 90% or above, and were collected. The cells were resuspended by a culture solution, and were counted under a microscope. The concentration of the cells was adjusted to 3.0×10⁴ cells/mL, and then, a cell suspension was seeded into a 96-well plate at a dose of 100 µL per well, and was incubated for 48 h at 37°C with 5% CO₂ in an incubator. In a dark environment, 10 µL of a CCK8 reagent was added to each well, followed by a water bath at 37°C in the dark for 4 h. A microplate reader was preheated 30 min in advance. After the incubation was completed, the absorbance at 450 nm per well was measured, and the data was analyzed. The experiment was independently repeated three times. The results obtained from the experiment were presented as mean ± SD, and Student's t-test was used for statistical analysis. **P*<0.05 was considered statistically significant, and ***P*<0.01 was considered extremely significant.

The results are as shown in FIG. 5. si-Gemin4 successfully silenced the expression of the *Gemin4* gene at the RNA and protein levels. Silencing of *Gemin4* gene significantly inhibited the proliferation of the cell proliferation of glioma cells U87, colorectal cancer cells HT29, and liver cancer cells HepG2 (FIG. 6), indicating that targeted inhibition of the *Gemin4* gene exerts an anti-tumor effect by inhibiting the proliferation of tumor cells.

### Example 4

This example provides the influence of silencing the expression of the *Gemin4* gene on the migration ability of tumor cells.

The three kinds of tumor cells (glioma cells U87, colorectal cancer cells HT29, and liver cancer cells HepG2) transfected with si-Gemin4 and si-NC in Example 3 were digested, counted and then seeded into a transwell chamber at 100 µL per well. Then, 0.6 mL of a complete culture medium containing 10% FBS was added to a transwell lower chamber to stimulate cell migration, and the cells were cultured at 37°C with 5% CO₂ for 48 h. The culture solution in the wells was discarded. Cells were fixed with methanol at room temperature for 30 min, stained with 0.1% crystal violet at room temperature for 10 min, and rinsed with clean water. Non-migrated cells at the upper layer were wiped off with a cotton swab. Migrated cells were observed under a microscope, with images captured from four randomly selected fields for counting. The experiment was independently repeated three times. The results obtained from the experiment were presented as mean ± SD, and Student's t-test was used for statistical analysis. **P*<0.05 was considered statistically significant, and ***P*<0.01 was considered extremely significant.

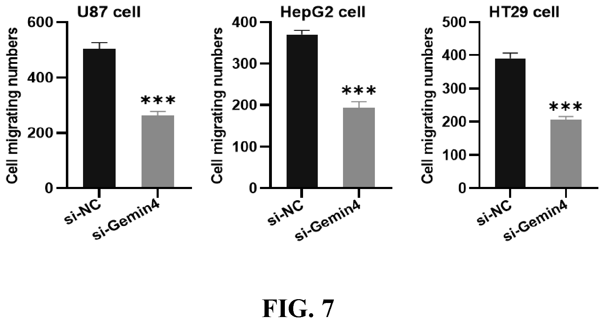

The results are as shown in FIG. 7. Silencing the expression of the *Gemin4* gene can significantly inhibit the migration of glioma cells U87, colorectal cancer cells HT29, and liver cancer cells HepG2, indicating that targeted inhibition of the *Gemin4* gene exerts an anti-tumor effect by inhibiting the migration of tumor cells.

The experiment designed in this application is scientifically reasonable, feasible and effective. Based on the above findings, the expression level of the *Gemin4* gene or the level of the Gemin4 protein can be used as a new biomarker to assist in the diagnosis of malignant tumors including gliomas, liver cancer, colorectal cancer, esophageal cancer, kidney cancer, breast cancer, gastric cancer, head and neck cancer, bile duct cancer, and lung cancer, and the *Gemin4* gene and/or Gemin4 protein can be directly used as treatment medicament for malignant tumors or used as potential targets for anti-tumor medicament. Therefore, the use of this gene and/or protein is greatly expanded, and new ideas and prospects are provided for medicament development in the future.

## Claims

1. Use of a *Gemin4* gene in preparation of a tumor detection reagent or a medicament for preventing or treating tumors, wherein the nucleic acid sequence of the gene is set forth in SEQ ID NO.1.

2. The use of a *Gemin4* gene in preparation of a tumor detection reagent or a medicament for preventing or treating tumors according to claim 1, wherein the tumor comprises one or more of human gliomas, liver cancer, colorectal cancer, esophageal cancer, kidney cancer, breast cancer, gastric cancer, head and neck cancer, bile duct cancer, and lung cancer.

3. The use of a *Gemin4* gene in preparation of a tumor detection reagent according to claim 2, wherein the tumor detection reagent comprises a specific primer pair with the nucleotide sequences set forth in SEQ ID NO.3 and SEQ ID NO.4.

4. The use of a *Gemin4* gene in preparation of a medicament for preventing or treating tumors according to claim 1 or 2, wherein the preventing or treating tumors comprises knockdown or silencing of the expression of the *Gemin4* gene in tumor cells.

5. The use of a *Gemin4* gene in preparation of a medicament for preventing or treating tumors according to claim 4, wherein the knockdown or silencing of the expression of the *Gemin4* gene comprises transfection of specific small interference RNA designed according to the nucleotide sequence of the *Gemin4* gene, and the nucleotide sequence of the specific small interference RNA is set forth in SEQ ID NO.5.

6. The use of a *Gemin4* gene in preparation of a medicament for preventing or treating tumors according to claim 4 or 5, wherein the preventing or treating tumors comprises inhibition of growth and/or metastasis of colorectal cancer, liver cancer and glioma cells.

7. Use of a Gemin4 protein in preparation of a tumor detection reagent, wherein the amino acid sequence of the Gemin4 protein is set forth in SEQ ID NO.2.

8. The use of a Gemin4 protein in preparation of a tumor detection reagent according to claim 7, wherein the tumor comprises one or more of human head and neck cancer, lung cancer, gastric cancer, bile duct cancer, breast cancer, kidney cancer, esophageal cancer, colorectal cancer, liver cancer, and gliomas.

9. A tumor detection reagent kit, comprising a reagent for detecting the expression level of a *Gemin4* gene, or a reagent for detecting the level of a Gemin4 protein, wherein the tumor comprises one or more of human gliomas, liver cancer, colorectal cancer, esophageal cancer, kidney cancer, breast cancer, gastric cancer, head and neck cancer, bile duct cancer, and lung cancer.

10. A pharmaceutical composition for preventing or treating a tumor, at least comprising a reagent for knocking down or silencing the expression of a *Gemin4* gene and a pharmaceutically acceptable carrier, wherein the tumor comprises one or more of gliomas, colorectal cancer, and liver cancer.
